(19) Europäisches Patentamt | European Patent Office | Office européen des brevets

(11) **EP 1 909 761 B1**

(12) **EUROPEAN PATENT SPECIFICATION**

(45) Date of publication and mention
of the grant of the patent:
**18.01.2017 Bulletin 2017/03**

(21) Application number: **06764269.4**

(22) Date of filing: **29.07.2006**

(51) Int Cl.:
*A61K 9/16* (2006.01)     *A61K 9/28* (2006.01)
*A61K 31/4439* (2006.01)

(86) International application number:
**PCT/EP2006/064824**

(87) International publication number:
**WO 2007/014928 (08.02.2007 Gazette 2007/06)**

(54) **PHARMACEUTICAL COMPOSITION COMPRISING GRANULAR PANTOPRAZOLE**

PANTOPRAZOL IN GRANULATFORM ENTHALTENDE PHARMAZEUTISCHE ZUSAMMENSETZUNG

PRÉPARATION PHARMACEUTIQUE COMPRENANT DU PANTOPRAZOLE GRANULAIRE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **29.07.2005 EP 05016555**

(43) Date of publication of application:
**16.04.2008 Bulletin 2008/16**

(73) Proprietor: **KRKA, tovarna zdravil, d.d., Novo mesto**
**8501 Novo mesto (SI)**

(72) Inventors:
• **VRECER, Franc**
**8351 Straza Pri Novem Mestu (SI)**
• **FERLAN, Andrej**
**1275 Smartno Pri Litiji (SI)**
• **KRAMAR, Andrejka**
**8000 Novo Mesto (SI)**
• **TURK, Urska**
**8000 Novo Mesto (SI)**
• **CERNOSA, Lidia**
**8311 Kostanjevica Na Krki (SI)**
• **BREZNIK, Marjanca**
**1000 Ljubljana (SI)**
• **RITLOP, Gregor**
**2000 Maribor (SI)**

(74) Representative: **Uexküll & Stolberg**
**Partnerschaft von**
**Patent- und Rechtsanwälten mbB**
**Beselerstraße 4**
**22607 Hamburg (DE)**

(56) References cited:
EP-A- 0 244 380          EP-A- 1 525 882
WO-A-00/40224           WO-A-2005/007117
WO-A-2005/032513         WO-A-2005/051348
WO-A2-2004/098594        US-A- 5 997 903
US-A1- 2004 052 832       US-A1- 2004 248 940

**Description**

**Field of the invention**

The present invention relates to pharmaceutical compositions which comprise granular pantoprazole as active ingredient and a method for the preparation of granular pantoprazole.

**Background of the invention**

Pantoprazole (5-(difluoromethoxy)-2{(3,4-di-methoxy-2-pyridinyl)-methyl]-sulphinyl}-H-benz-imidazole) is a proton pump inhibitor belonging to the group of benzimidazoles. This compound inhibits gastric acid formation and thereby it is very efficient for the treatment of gastric and duodenum ulcers, gastro-oesophageal reflux and the like. It inhibits gastric acid secretion by inhibiting $H^+$-$K^+$ ATPase (proton-pump) activity. Pantoprazole and physiologically acceptable salts thereof are known from EP 0 166 287 and Kohl et al., The Journal of Medicinal Chemistry; 1992, 35, No. 6, 1049-1057.

Pantoprazole is known to be sensitive to heat and unstable in an acidic environment as well as in the presence of moisture and organic solvents. Thus, pharmaceutical compositions comprising this drug, as well as processes for the manufacture thereof, must be designed to protect the drug from moisture and acidic conditions. Due to the rapid drug degradation that occurs in acidic gastric fluids, such formulations usually comprise an enteric coating.

The stability problems associated with benzimidazole compounds are well known in the art, which teaches various measures for preparing stable formulations containing benzimidazole compounds. The most common approach is the use of an alkaline core, a separating layer and an enteric coating. By use of an alkaline substance within the core benzimidazole compounds can be protected against acid degradation.

EP 0 244 380 discloses pharmaceutical preparations containing acid labile compounds. The preparations consist of a core containing either the active ingredient together with an alkaline reacting compound or an alkaline salt of the active ingredient, one or more water-soluble sub-coating layers which may also contain an alkaline component and an enteric coating layer.

According to WO 92/22284 certain ingredients such as lactose, microcrystalline cellulose and hydroxypropyl cellulose being present in the compositions of EP 0 244 380 accelerate the decomposition of the pantoprazole. It is said that more stable formulations could be obtained by using polyvinylpyrrolidone and/or hydroxypropyl-methyl cellulose as binder and mannitol as an optional inert filler in the tablet core.

WO 2005/051348, on the other hand, discloses that hygroscopic polymeric binders such as polyvinylpyrrolidone impair the release of the active ingredient after storage due to absorbed humidity. It was found that by using appropriately chosen excipients compatible with the active ingredient the use of a polymeric binder can be avoided. This document teaches the use of tablet cores containing pantoprazole as active ingredient, spray-dried or granulated mannitol having an average particle size between 100 $\mu$m and 500 $\mu$m, an alkalizing substance, a disintegrant and a lubricant. The active ingredient can be granulated with an aqueous solution of mannitol in order to improve its flowability. It is known that drying of granulates based on sugar alcohols is difficult and time consuming and requires a high energy input. Furthermore, prolonged drying times are disadvantageous in view of the heat sensitivity of pantoprazole.

**Description of the Invention**

It is the object of the present invention to provide tablets according to claim 1 comprising pantoprazole as the active ingredient which are stable and which can be prepared by a direct compression process.

It was found by the present inventors that this object can be achieved by using pantoprazole in the form of particles having an average particle size of 60 to 250 $\mu$m, preferably 100 to 250 $\mu$m, more preferably 125 to 250 $\mu$m, even more preferably 125 to 230 $\mu$m and most preferably 140 to 200 $\mu$m. If pantoprazole having average particle size in the range 60 to 250 $\mu$m is mixed with other ingredients such as alkalizing substances, fillers and disintegrants, the mixture can easily be formed into tablet cores by direct compression thereof without the addition of water. Pantoprazole which is commercially available may have an average particle size of as little as 25 $\mu$m.

This finding is surprising since pantoprazole exhibits inappropriate physical properties for direct compression. Furthermore, the tablet cores usually contain 20 to 50 % by weight, preferably 25 to 30 % by weight of pantoprazole. Due to the relatively high amount of the active ingredient the addition of other excipients which are better suited for direct compression is limited. If the size of the tablet cores exceeds a certain weight the disintegration of the tablets within the small intestine would be delayed and the pharmacological effect of the active ingredient would be diminished.

The term "average particle size" as used herein refers to the volume mean diameter of particles. The volume mean diameter can be determined by laser light scattering using e.g. a Malvern-Mastersizer Apparatus MS 2000. Particle sizes are determined by measuring the angular distribution of laser light scattered by a homogeneous suspension of particles. The size distribution is determined from the light scattering data using the theory of light scattering developed by Gustav Mie.

The pantoprazole particles consist of pantoprazole or pharmaceutically acceptable a salt thereof.

The compositions according to the invention contain as active ingredient pantoprazole, preferably in the form of a pharmaceutically acceptable salt thereof such as the sodium salt of pantoprazole, more preferably the sesquihydrate of the sodium salt of pantoprazole.

The compositions have the form of a tablet comprising

(a) a core comprising the active ingredient, an alkalizing substance, a filler and a disintegrant;
(b) an optional water-soluble separating layer or subcoating; and
(c) an enteric coating having a thickness of 85$\mu$m to 150$\mu$m.

An additional water-soluble protecting layer may be applied to the enteric coating layer.

The tablets according to the invention contain as the alkalizing agent preferably an alkali or earth alkali hydroxide, an alkali or earth alkali carbonate, an alkali or earth alkali hydrogen carbonate, an alkali phosphate, such as sodium phosphate, a glucosamine or a mixture of two or more of these compounds, preferably sodium hydroxide, sodium carbonate, sodium hydrogen carbonate, or a mixture of two or more of these components, more preferably sodium carbonate.

As filler the tablets preferably contain lactose or a sugar alcohol, such as mannitol, sorbitol, maltitol, erythrol, or a mixture of two or more of these components. The most preferred fillers are mannitol and sorbitol and mixtures thereof. It is preferred that the filler has an average particle size of 30 to 500 $\mu$m, more preferably an average particle size of 250 to 500 $\mu$m. Mannitol is known for its non-hygroscopicity while sorbitol which is an isomer of mannitol is hygroscopic. Mixtures of mannitol and sorbitol proved to be particularly suitable as a filler. This finding is surprising since hygroscopic additives are said to impair the release of pantoprazole after storage.

Tablets prepared according to the present invention did not show a significant decrease in the dissolution of the active ingredient after storage even if sorbitol was used as the only filler. It is preferred to use mixtures comprising 1 part by weight of mannitol and 1 to 5 parts by weight of sorbitol, more preferably 2 to 4 parts by weight of sorbitol and most preferably 2.8 to 3.8 parts by weight of sorbitol.

Preferred disintegrants are crospovidone, sodium starch glycolate, cross-linked carboxylic methylcelluloses, such as croscarmelose, sodium, sodium carboxymethyl starch, sodium carboxymethyl cellulose, potassium carboxymethyl cellulose, or a mixture of two or more of these components. Crospovidone is the most preferred disintegrant. Crospovidone is cross-linked polyvinyl pyrrolidone.

Pantoprazole containing tablets should disintegrate rapidly when leaving the stomach and reaching the upper part of the intestine where the physiological pH raises to values higher than 5.5 and where the polymer of the enteric coating dissolves. It has surprisingly been found that the disintegration of coated tablets can be improved by using a disintegrant having a defined particles size. By using a combination of disintegrant with an average particle size 10 to 60 $\mu$m (Type A), preferably crospovidone, and disintegrant with an average particle size 80 to 200 $\mu$m (Type B), preferably crospovidone, the disintegration time can be significantly reduced. Mixtures comprising 1 part by weight of disintegrant Type A and 1 to 5 parts by weight of disintegrant Type B, preferably 2 to 4 parts by weight of disintegrant of Type B proved to be particularly advantageous.

The compositions of the present invention preferably release more than 50 % of the active ingredient within 45 minutes and more than 80 % of the active ingredient within 60 minutes, determined in phosphate buffer of pH 6.4, USP apparatus 1, 100 rpm, after storage in 0.1 M HCL solution of pH 1 for 1 hour.

The tablet cores preferably also contain a lubricant. Preferred lubricants are stearic acid, hydrogenated vegetable oils, paraffin or alkali or alkaline earth stearates, aluminum stearate, sodium stearyl fumarate or mixtures of two or more of these compounds. The most preferred lubricants are magnesium stearate and calcium stearate.

The tablet cores may also comprise a binder, such as microcrystalline cellulose, hydroxypropyl methyl cellulose, polyvinylpyrrolidone (povidone) or mixtures of two or more of these compounds.

The tablet cores preferably comprise

15 to 50 % by weight, preferably 20 to 30 % by weight, more preferably 25 to 30 % by weight of the active ingredient,

2 to 25 % by weight, preferably 5 to 20 % by weight, more preferably 5 to 15 % by weight of the alkalizing substance,

10 to 50 % by weight, preferably 20 to 40 % by weight, more preferably 25 to 35 % by weight of the filler, and

10 to 50 % by weight, preferably 20 to 40 % by weight, more preferably 25 to 40 % by weight of the disintegrant.

According to a preferred embodiment the film-coated tablet according to the invention contain on the surface of the tablet core a sub-coating or separating layer. The optional water-soluble separating layer applied on the tablet core may contain film-forming substances generally used for the preparation of film-coated tablets. A preferred water-soluble film-forming substance is hydroxypropyl-methyl cellulose alone or in combination with other water soluble polymers such as polyvinylpyrolidone. Besides the film-forming polymer optionally further auxiliary agents, such as plasticizers, e.g. polyethylene glycol, propylene glycol, titanium dioxide and talc can be used in the coating. The sub-coating is used to separate gastro-resistant coating from direct contact with tablet core.

The sub-coating is preferably applied in an amount of 2 to 20 % by weight, preferably 5 to 15 % by weight (related to the total weight of the composition).

The optional separating layer has preferably a thickness of 20 to 120 $\mu$m, more preferably of 50 to 90 $\mu$m.

The enteric coating layer preferably comprises film-forming substances such as cellulose acetate phthalate, polyvinylacetate phthalate, hydroxypropyl-methyl cellulose phthalate, hydroxypropyl-methyl cellulose acetate succinate, ethylacrylate methacrylic acid copolymer, methylmethacrylate methacrylic acid copolymer, shellac, more preferably copolymers of methacrylic acid and methacrylic acid esters, such as anionic polymer of methacrylic acid and methacrylates with -COOH groups, e.g. L-form Eudragits. These polymers can be used as organic solutions or preferably as aqueous dispersions, and optionally plasticizers, e.g. triacetin, polyethyleglycole (macrogol) or triethyl citrate, or talc may be added to them. The enteric coating is insoluble in acidic medium. It prevents a disintegration of the tablets in the acidic medium of the stomach which would cause degradation of the pantoprazole and consequently diminishing of therapeutic effect of such the composition.

The enteric coating layer is preferably applied in an amount of 8 to 20 % by weight, more preferably 10 to 15 % by weight (related to the total weight of the composition).

The enteric coating layer has preferably a thickness of 90 to 120 $\mu$m.

The tablets preferably comprise 20 to 40 mg, in particular 20 mg or 40 mg of pantoprazole, calculated as pure pantoprazole. This means that e.g. 20 mg or 40 mg of pure pantoprazole are incorporated into each tablet. If pantoprazole is used in the form of a salt and or a hydrate, the mass of the active ingredient which is actually used is calculated such that it corresponds to 20 to 40 mg of pure pantoprazole. According to a preferred embodiment of the invention the enteric coating of these tablets has the same thickness even if the tablets differ in strengths, size or quantities of ingredients. The same thickness means, that the thickness of the enteric coating layer of the tablets does not differ by more than 5 % measured by SEM on the cross-section of the tablets. If the surface area of the cores of the tablets is not the same the quantity of enteric coating material which is applied to the cores is adjusted such that the same thickness of the enteric coating layer is achieved.

The thickness of the enteric coating layer of different tablets can be adjusted such that the same functionality is achieved. The functionality of enteric coating layers for different tablets is determined by comparing the results of dissolution testing of the active ingredient (% of released pantoprazole in phosphate buffer solution, pH 6.8, USP Apparatus 2, 50 rpm, at 37°C; tablets are first exposed for two hours to 0.1 M solution of HCl and then the medium is exchanged for phosphate buffer solution of pH 6.8; similarity factors for the dissolution profiles of different tablets can then be calculated and compared). The similarity of the dissolution profiles is compared by calculating the similarity factor (according to the EMEA guideline CPMP/QWP/604/96 NOTE FOR GUIDANCE ON QUALITY OF MODIFIED RELEASE PRODUCTS: A: ORAL DOSAGE FORMS B: TRANSDERMAL DOSAGE FORMS SECTION I (QUALITY)).

The similarity factor is defined as follows:

$$\texttt{f}_2 \;=\; 50 \;\texttt{x}\; \log\{[1+(1/n)\Sigma_{t=1}{}^{n}(R_t-T_t)^2]^{-0.5}\;\texttt{x}\;100\}$$

In this equation $f_2$ is the similarity factor, n is the number of time points, R(t) is the mean percent drug dissolved of e.g. the current formulation, and T(t) is the mean percent drug dissolved of e.g. the changed composition.

The evaluation of similarity is based on the condition of (i) a minimum of three time points, (ii) 12 individual values for every time point, (iii) not more than one mean value of > 85 % dissolved; (iv) that the standard deviation of the mean should be less than 10 % from the second to last time point. An $f_2$ value between 50 and 100 suggests that two dissolution profiles are similar.

The thickness of the sub-coating and the enteric coating is measured on the scanning electron microscope of the cross-section of coated tablets.

The film-coated tablet according to the invention containing pantoprazole can be prepared as follows. First a tablet core is provided by mixing of the active ingredient, a disintegrant, a filler, an alkalizing agent and any optional components, and compressing the homogenous mixture to tablets without the addition of water. Mixing is preferably performed in a biconical mixer and tablets are preferably formed on a rotary tabletting machine. The thus-obtained tablets are then coated first with a water-soluble coating, then with an enteric coating resistant to gastric acid and finally with a further optional water-soluble coating.

The compositions can optionally be subjected to a curing step. The curing step is preferably performed after application of the dispersion of the acid insoluble polymer (gastro resistant coating). The curing step is preferably performed in the coating equipment such as a perforated coating pan known in the state of the art (e.g. Manesty types, Accela Cota, GS coating pan or Glatt coater) and/or in a drying chamber. Curing is preferably performed at a composition temperature of 30 to 50 °G, more preferably 35 to 45 °C, and the preferred curing time is in the range of 1 to 16 hours, more preferably 2 to 8 hours.

The pantoprazole particles used for preparing the pharmaceutical compositions are produced by agglomeration of smaller particles which are commercially available. Agglomeration is performed by wetting pantoprazole with water, passing the wet pantoprazole through a sieve having a mesh size in the range 0.8 to 2 mm, preferably 0.8 to 1.2 mm, more preferably 1.0 mm, and drying the mass in a fluid bed dryer preferably at product temperatures 35 to 50°C. The order of the sieving step and the drying step is optional.

The water is preferably adjusted to a pH of 10 to 12 by the addition of an alkalizing agent such as sodium hydroxide, sodium carbonate, trisodium phosphate, meglumine or glucosamine. Mixing is preferably performed in a high shear mixer. As the starting material commercially available pantoprazole can be used, preferably pantoprazole having an average particle size of at least 25 $\mu$m is used. Drying is preferably performed at a temperature of 35 to 50°C.

According to the process of the present invention granulation of the active ingredient can be achieved without the addition of other ingredients such as sugar alcohols and disintegrants. The granulate of the present invention can be dried in significantly shorter time than granulate prepared according to the prior art e.g. by use of an aqueous solution of mannitol. A granulate of agglomerated pantoprazole sodium sesquihydrate prepared according to the present invention can be dried to a residual moisture content below 1 % by weight in less than 1 hour while the moisture content of a granulate prepared with an aqueous solution of mannitol is just below 3 % by weight after 2 to 3 hours of drying at identical conditions. The residual moisture is determined by a halogen moisture analyzer (Mettler HR 73; 20 min at 85 °C). Thus the present invention provides a granulate containing less moisture than prior art granulates, preferably less than 1 % by weight, and less degradation products since drying of prior art granulates down to a moisture content of less than 1 % by weight unavoidably results in the formation of degradation products. Furthermore, due to the reduced moisture content the granulate shows improved stability during storage. The agglomerated drug can be used in the preparation of tablet cores either by direct compression or further granulation with excipients such as fillers, stabilizers and disintegrants.

**Examples**

***Example 1: Preparation of agglomerated pantoprazole sodium sesquihydrate***

6.14 kg of pantoprazole sodium sesquihydrate were transferred into the high shear mixer (Collet Gral 75) and wetted while mixing with 1.9 kg purified water. The wet mass was transferred to a fluid bed dryer (Glatt WSG 5) and dried with inlet air temperature (60°C) for 25 minutes. After drying the granulate was sieved through a 1.0 mm sieve and dried for an additional 20 minutes. The residual moisture of the dried granulate was 0.6% as determined by a halogen moisture analyzer (Mettler HR 73; 20 min at 85°C).

The average particle size of the product was determined by laser light scattering. 100 to 800 mg of the particles were dispersed in 5 to 8 ml of vegetable oil (sunflower oil). No solubilizers or surfactants were used. Then the obtained suspension was subjected to size determination (Malvern Mastersizer MS 2000). According to the manufacturer's information, the Malvern Mastersizers allows for a measurement of particle size distributions in the range of 20 nm to 2000 $\mu$m, with a precision of better than 0.5%. The product had an average particle size of 141 $\mu$m. This product was used in the following examples.

***Example 2:***

A) Tablet core

| Ingredient | Amount |
|---|---|
| Pantoprazole sodium sesquihydrate (Example 1) | 315.7 g |
| Sodium carbonate | 140.0 g |

(continued)

| Ingredient | Amount |
|---|---|
| Mannitol (granulated) | 153.3 g |
| Crosspovidone (type A)[1] | 350.0 g |
| Microcrystalline cellulose (Avicel PH 200) | 245.0 g |
| Magnesium stearate | 21.0 g |

[1] cross-linked polyvinyl pyrrolidone, average particle size < 60 $\mu$m

All ingredients were mixed in biconical mixer and pressed into tablets on rotary tabletting machine.

B) Separating layer

| Ingredient | Amount |
|---|---|
| Hypromellose[1] 3 cp | 176.0 g |
| Kollidon K25[2] | 4.4 g |
| Propylenglycole | 44.0 g |
| Water | 1375.0 g |

[1] hydroxypropyl methyl cellulose
[2] polyvinylpyrrolidone (not cross-linked)

Hypromellose and Kollidon were dissolved in water. Propylenglycole was added to the solution. Tablet cores obtained under A were coated by spraying the solution obtained under B onto the tablet cores using a Manesty coating equipment.

C) Enteric coating

| Ingredient | Amount |
|---|---|
| Eudragit L-30D[1] | 176.0 g[2] |
| Triethycitrate | 17.6 g |
| Water | 583.0 g |

[1] anionic polymer of methacrylic acid and methacrylates with a -COOH group
[2] expressed as dry substance weight

Eudragit dispersion was diluted with water and triethylcitrate was dispersed into the diluted Eudragit dispersion. The coating dispersion containing Eudragit L30D, triethylcitrate and water was sprayed onto coated tablets obtained under B using the same equipment as in step B.

*Example 3:*

A) Tablet core

| Ingredient | Amount |
|---|---|
| Pantoprazole sodium sesquihydrate (Example 1) | 315.7 g |
| Sodium carbonate | 140.0 g |
| Lactose (spray dryed) | 153.3 g |

(continued)

| Ingredient | Amount |
|---|---|
| Crosspovidone (Type B) | 350.0 g |
| Microcrystalline cellulose (Avicel PH 200) | 245.0 g |
| Magnesium stearate | 21.0 g |

All ingredients were mixed in biconical mixer and pressed into tablets on rotary tabletting machine.

B) Separating layer

| Ingredient | Amount |
|---|---|
| Hypromellose 3 cp | 176.0 g |
| Kollidon K25 | 4.4 g |
| Propylenglycole | 44.0 g |
| Water | 1375.0 g |

Hypromellose and Kollidon were dissolved in water. Propylenglycole was added to the solution. Tablet cores obtained under A were coated by spraying the solution obtained under B onto the tablet cores in a Manesty coating equipment.

C) Enteric coating

| Ingredient | Amount |
|---|---|
| Eudragit L-30D | 211.2 g[1] |
| Macrogole[2] 6000 | 21.1 g |
| Talc | 79.2 g |
| Water | 700.0 g |

[1] expressed as dry substance weight
[2] polyethylenglycol

Eudragit dispersion was diluted with a part of the water and Macrogole was dissolved in rest of water. Talc was dispersed into the Macrogole solution and the obtained suspension was added to the diluted Eudragit dispersion. The obtained suspension was sprayed onto coated tablets obtained under B using the same equipment as in step B.

*Example 4:*

A) Tablet core

| Ingredient | Amount |
|---|---|
| Pantoprazole sodium sesquihydrate (Example 1) | 315.7 g |
| Sodium carbonate anhydrous | 140.0 g |
| Mannitol | 363.3 g |
| Crosspovidone (type A)[1] | 70.0 g |
| Crosspovidone (type B)[2] | 280.0 g |

(continued)

| Ingredient | Amount |
|---|---|
| Magnesium stearate | 21.0 g |

[1] cross-linked polyvinyl pyrrolidone, average particle size < 60 $\mu$m
[2] cross-linked polyvinyl pyrrolidone, average particle size > 80 $\mu$m

All ingredients were mixed in biconical mixer and pressed into tablets on rotary tabletting machine. Loss on drying of the mixture for tabletting: 1,75% (Mettler halogen dryer: 85°C 20 min.)

B) Separating layer

| Ingredient | Amount |
|---|---|
| Hypromellose 3 cp | 176.0 g |
| Kollidon K25 | 4.4 g |
| Titanium dioxide | 3.7 g |
| Propylenglycole | 44.0 g |
| Water | 1375.0 g |

Hypromellose and Kollidon were dissolved in water. Propylenglycol was added to the solution. Pigment was homogeneously dispersed into the obtained solution. Tablet cores obtained under A were coated by spraying the suspension obtained under B onto the tablet cores in a Manesty coating equipment.

C) Enteric coating

| Ingredient | Amount |
|---|---|
| Eudragit L-30D | 125.4 g[1] |
| Triethycitrate | 12.5 g |
| Macrogole 6000 | 2.6 g |
| Talc | 57.4 g |
| Water | 415.8 g |

[1] expressed as dry substance weight

Eudragit dispersion was diluted with a 1st part of water and Macrogole was dissolved in 2nd part of water. Talc was dispersed into the Macrogole solution and the obtained suspension was added to the diluted Eudragit dispersion. Trietylcitrate was dispersed in 3rd part of water and the obtained dispersion was added to the suspension. The obtained coating suspension was sprayed onto coated tablets obtained under B using the same equipment as in step B.

***Example 5:***

A) Tablet core

| Ingredient | Amount |
|---|---|
| Pantoprazole sodium sesquihydrate (Example 1) | 315.7 g |
| Sodium carbonate anhydrous | 140.0 g |
| Magnesium carbonate heavy | 14.0 g |

(continued)

| Ingredient | Amount |
|---|---|
| Sorbitole (granulated) | 349.3 g |
| Crosspovidone (Type B) | 350.0 g |
| Magnesium stearate | 21.0 g |

All ingredients were mixed in biconical mixer and pressed into tablets on rotary tabletting machine.

B) Separating layer

| Ingredient | Amount |
|---|---|
| Hypromellose 3 cp | 176.0 g |
| Kollidon K25 | 4.4 g |
| Titanium dioxide | 3.7 g |
| Propylenglycole | 44.0 g |
| Water | 1375.0 g |

Hypromellose and Kollidon were dissolved in water. Propylenglycol was added to the solution. Pigment was homogeneously dispersed into the obtained solution. Tablet cores obtained under A were coated by spraying the suspension obtained under B onto the tablet cores in a Manesty coating equipment.

C) Enteric coating

| Ingredient | Amount |
|---|---|
| Eudragit L-30D | 146.3 g[1] |
| Triethycitrate | 14.6 g |
| Macrogole 6000 | 3.1 g |
| Talc | 67.0 g |
| Water | 485.0 g |

[1] expressed as dry substance weight

Eudragit dispersion was diluted with a 1st part of the water and Macrogole was dissolved in 2nd part of water. Talc was dispersed into the Macrogole solution and the obtained suspension was added to the diluted Eudragit dispersion. Trietylcitrate was dispersed in 3rd part of water and the obtained dispersion was added to the suspension. The obtained coating suspension was sprayed onto coated tablets obtained under B using the same equipment as in step B.

Example 6:

A) Tablet core

| Ingredient | Amount |
|---|---|
| Pantoprazole sodium sesquihydrate (Example 1) | 631,4 g |
| Sodium carbonate anhydrous | 140,0 g |
| Sorbitole (granulated) | 518,0 g |
| Crospovidone (Type A) | 210,0 g |

(continued)

| Ingredient | Amount |
|---|---|
| Crospovidone (Type B) | 700,0 g |
| Calcium stearate | 14.0 g |

All ingredients were mixed in biconical mixer and pressed into tablets on rotary tabletting machine.

B) Separating layer

| Ingredient | Amount |
|---|---|
| Hypromellose 3 cp | 176.0 g |
| Kollidon K25 | 4.4 g |
| Titanium dioxide | 3.7 g |
| Propylenglycole | 44.0 g |
| Water | 1375.0 g |

Hypromellose and Kollidon were dissolved in water. Propylenglycol was added to the solution. Pigment was homogeneously dispersed into the obtained solution. Tablet cores obtained under A were coated by spraying the suspension obtained under B onto the tablet cores in a Manesty coating equipment.

C) Enteric coating

| Ingredient | Amount |
|---|---|
| Eudragit L-30D | 176,0 g[1] |
| Macrogole 6000 | 17,6 g |
| Talc | 66.0 g |
| Water | 583.0 g |

[1] expressed as dry substance weight

Eudragit dispersion was diluted with a 1st part of the water and Macrogole was dissolved in 2nd part of water. Talc was dispersed into the Macrogole solution and the obtained suspension was added to the diluted Eudragit dispersion. The obtained coating suspension was sprayed onto coated tablets obtained under B using the same equipment as in step B. After the coating suspension had been sprayed onto the coated tablets, the product was transferred to a drying chamber and kept at 40 °C for 8 hours.

***Exampl e 7:***

A) Tablet core

| Ingredient | Amount |
|---|---|
| Pantoprazole sodium sesquihydrate[1] | 315.7 g |
| Sodium carbonate anhydrous | 70.0 g |
| Crospovidone (Type A) | 105.0 g |
| Mannitol (kristalline) | 83.3 g |

[1] average particle size 66.0 $\mu$m

0.3 g sodium carbonate anhydrous was dissolved in 370 g of water to form solution (I). The ingredients listed in the above table were placed into a high shear mixer (Collet grall 10) and solution (I) was slowly added to the mixture while mixing. Kneading was used for 1 min. The wet mass was passed through 18 mesh (1.0 mm) sieve and dried in fluid bed granulator/dryer (Glatt GPCG-3) until the loss on drying was lower than 3%.

To the dry granulate following ingredients were added to obtain a mixture for tabletting:

| Ingredient | Amount |
|---|---|
| Sorbitole (granulated) | 259,0 g |
| Crosspovidone (Type B) | 350.0 g |
| calcium stearate | 7.0 g |

All ingredients were mixed in biconical mixer and pressed into tablets on rotary tabletting machine.

B) Separating layer

| Ingredient | Amount |
|---|---|
| Hypromellose 3 cp | 176.0 g |
| Kollidon K25 | 4.4 g |
| Titanium dioxide | 3.7 g |
| Propylenglycole | 44.0 g |
| Water | 1375.0 g |

Hypromellose and Kollidon were dissolved in water. Propylenglycol was added to the solution. Pigment was homogeneously dispersed into the obtained solution. Tablet cores obtained under A were coated by spraying the suspension obtained under B onto the tablet cores in a Manesty coating equipment.

C) Enteric coating

| Ingredient | Amount |
|---|---|
| Eudragit L-30D | 176,0 g[1] |
| Macrogole 6000 | 17,6 g |
| Talc | 66.0 g |
| Water | 583.0 g |
| [1] expressed as dry substance weight | |

Eudragit dispersion was diluted with a 1st part of water and Macrogole was dissolved in 2nd part of water. Talc was dispersed into the Macrogole solution and the obtained suspension was added to the diluted Eudragit dispersion. The obtained coating suspension was sprayed onto coated tablets obtained under B using the same equipment as in step B. The coated tablets produced in step C were then kept in the coating equipment (Manesty XL) at 45 °C for 4 hours at periodical slow revolution of the pan (1 revolution every 5 minutes) in order to diminish mechanical stress on the tablets and to achieve good temperature distribution in the tablet mass in the pan.

*Example 8:*

A) Tablet core

| Ingredient | Amount |
|---|---|
| Pantoprazole sodium sesquihydrate (Example 1) | 315.7 g |
| Sodium carbonate anhydrous | 70.0 g |
| Crospovidone (TypeA) | 105.0 g |
| Mannitol (kristalline) | 83.3 g |

0.3 g Anhydrous sodium carbonate was dissolved in 370 g of water to form solution (I). The ingredients listed in the above table were placed into a high shear mixer (Collet grall 10) and solution (I) was slowly added to the mixture while mixing. Kneading was used for 1 min. The wet mass was passed through 18 mesh (1.0 mm) and dried in fluid bed dryer (Glatt GPCG-3) for 3 hours to a residual moisture content of 3% (Halogen moisture analyzer Mettler HR 73; 20 min at 85°C).

To the dry granulate following ingredients were added to obtain a mixture for tabletting:

| Ingredient | Amount |
|---|---|
| Sorbitole (granulated) | 259,0 g |
| Crosspovidone (Type B) | 350.0 g |
| calcium stearate | 7.0 g |

All ingredients were mixed in biconical mixer and pressed into tablets on rotary tabletting machine.

B) Separating layer

| Ingredient | Amount |
|---|---|
| Hypromellose 3 cp | 176.0 g |
| Kollidon K25 | 4.4 g |
| Titanium dioxide | 3.7 g |
| Propylenglycole | 44.0 g |
| Water | 1375.0 g |

Hypromellose and Kollidon were dissolved in water. Propylenglycole was added to the solution. Pigment was homogeneously dispersed into the obtained solution. Tablet cores obtained under A were coated by spraying the suspension obtained under B onto the tablet cores in a Manesty coating equipment.

C) Enteric coating

| Ingredient | Amount |
|---|---|
| Eudragit L-30D | 176,0 g[1] |
| Macrogole 6000 | 17,6 g |
| Talc | 66.0 g |
| Water | 583.0 g |
| [1] expressed as dry substance weight | |

Eudragit dispersion was diluted with a 1st part of water and Macrogole was dissolved in 2nd part of water. Talc was dispersed into the Macrogole solution and the obtained suspension was added to the diluted Eudragit dispersion. The obtained coating suspension was sprayed onto coated tablets obtained under B using the same equipment as in step B.

*Example 9:*

A) Tablet core

| Ingredient | Amount for 20 mg tablets | Amount for 40 mg tablets |
|---|---|---|
| Pantoprazole sodium sesquihydrate | 157.85 g | 315.7 g |
| Sodium carbonate anhydrous | 35.00 g | 70.0 g |
| Crospovidone (Type A) | 52.50 g | 105.0 g |
| Mannitol (kristalline) | 41.65 g | 83.3 g |

Procedure of granulation is the same as in example 7.

| Ingredient | Amount for 20 mg tablets | Amount for 40 mg tablets |
|---|---|---|
| Sorbitole (granulated) | 126.0 g | 252,0 g |
| Crosspovidone (Type B) | 175.0 g | 350.0 g |
| calcium stearate | 7.0 g | 14.0 g |

All ingredients were mixed in biconical mixer and pressed into tablets on rotary tabletting machine.

B) Separating layer

| Ingredient | Amount for 20 mg tablets* | Amount for 40 mg tablets* |
|---|---|---|
| Hypromellose 3 cp | 56.91 g | 113.82 g |
| Kollidon K25 | 1.40 g | 2.80 g |
| Titanium dioxide | 1.05 g | 2.10 g |
| Colouring agent E172 | 0.14 g | 0.28 g |
| Propylenglycole | 14.00 g | 28.00 g |
| Water | 444.60 g | 889.20 g |
| * overage of the suspensions was used due to losses during coating | | |

Hypromellose and Kollidon were dissolved in water. Propylenglycol was added to the solution. Pigment was homogeneously dispersed into the obtained solution. Tablet cores obtained under A were coated by spraying the suspension obtained under B onto the tablet cores in a Manesty coating equipment.

C) Enteric coating

| Ingredient | Amount for 20 mg tablets[2] | Amount for 40 mg tablets[2] |
|---|---|---|
| Eudragit L-30D | 70.00 g[1] | 112.00 g[1] |
| Macrogole 6000 | 7.00 g | 11.20 g |
| Talc | 25.90 g | 42.00 g |
| Water | 235.00 g | 375.00 g |
| [1] expressed as dry substance weight | | |
| [2] overage of the suspensions was used due to losses during coating | | |

Eudragit dispersion was diluted with a 1$^{st}$ part of water and Macrogole was dissolved in 2$^{nd}$ part of water. Talc was dispersed into the Macrogole solution and the obtained suspension was added to the diluted Eudragit dispersion. The obtained coating suspension was sprayed onto coated tablets obtained under B using the same equipment as in step B. The coated tablets produced in step C were kept in the coating equipment (Manesty XL) at 40 °C for 4 hours at periodical slow revolution of the pan (1 revolution every 5 minutes) in order to diminish mechanical stress on the tablets and to achieve good temperature distribution in the tablet mass in the pan.

The gastroresistance and similarity of dissolution profiles of the 20 mg and 40 mg pantoprazole tablets was determined. in 0.1 M HCl, 500 ml, USP apparatus 1 (baskets), 100 rpm, 2 hours. Assay of intact pantoprazole in tablets after 2 hours in 0.1 M HCL was determined by HPLC method and expressed as % of declared amount.

Dissolution of pantoprazole for calculating the similarity factor in buffer stage was determined using phosphate buffer pH 6.8, 900 ml, USP apparatus 1 (baskets), 100 rpm, after acidic stage (0.1 M HCl, 500 ml, USP apparatus 1 (baskets), 100 rpm, 2 hours). % of release of pantoprazole was followed up to 60 minutes and determined by UV method.

| Tablet | Gastro-resistance | Similarity factor (buffer stage) |
|---|---|---|
| 40 mg tablets | 102-106%; avg: 104%* | 68.1 |
| 20 mg tablets | 99-104%; avg: 100%* | |
| * average value of 6 determinations | | |

**Claims**

1. Pharmaceutical composition comprising pantoprazole as the active ingredient having the form of a tablet comprising

   (a) a core comprising the active ingredient, an alkalizing substance, a filler and a disintegrant;
   (b) an optional water-soluble separating layer; and
   (c) an enteric coating,

   **characterized in that** the enteric coating layer has a thickness of 85 $\mu$m to 150 $\mu$m and **in that** the pantoprazole is present in the form of particles having an average particle size within the range of 60 $\mu$m to 250 $\mu$m, which particles consist of pantoprazole and are obtainable by a process comprising the steps of wetting pantoprazole with water in a high shear mixer, passing the wet pantoprazole through a sieve having a mesh size within the range of 0.8 to 2 mm, and drying the mass in a fluid bed dryer, the order of the sieving and drying step being optional.

2. The pharmaceutical composition of claim 1, wherein the pantoprazole particles have an average particle size within the range of 100 $\mu$m to 250 $\mu$m.

3. The composition of claim 1 or 2, wherein the pantoprazole particles have an average particle size within the range of 125 $\mu$m to 230 $\mu$m.

4. The composition of any one of the preceding claims, wherein the pantoprazole particles have an average particle size within the range of 140 $\mu$m to 200 $\mu$m.

5. The composition of any one of the preceding claims, wherein the pantoprazole is present in form of the sodium salt.

6. The composition of claim 5, wherein the pantoprazole is present in the form of pantoprazole sodium sesquihydrate.

7. The composition of any one of the preceding claims, wherein the alkalizing agent is an alkali or earth alkali hydroxide, an alkali or earth alkali carbonate, an alkali or earth alkali hydrogen carbonate, a phosphate, a glucosamine or a mixture of two or more of these compounds, preferably sodium hydroxide, sodium carbonate, sodium hydrogen carbonate, sodium phosphate or a mixture of two or more of these components, more preferably sodium carbonate.

8. The composition of any one of the preceding claims, wherein the filler is lactose or a sugar alcohol, such as mannitol, sorbitol, maltitol, erythrol, or a mixture of two or more of these components.

9. The composition of claim 8, wherein the filler is a mixture of mannitol and sorbitol.

10. The composition of claim 9, wherein the mixture comprises 1 part by weight of mannitol and 1 to 5 parts by weight of sorbitol, preferably 2 to 4 parts by weight of sorbitol and more preferably 2.8 to 3.8 parts by weight of sorbitol

11. The composition of any one of claims 8 to 10, wherein the sugar alcohol has an average particle size within the range of 30 $\mu$m to 500 $\mu$m.

12. The composition of any one of the preceding claims, wherein the disintegrant is crospovidone, sodium starch glycolate, croscarmelose sodium, sodium carboxymethyl starch, sodium carboxymethyl cellulose, potassium carboxymethyl cellulose, or a mixture of two or more of these components.

13. The composition of claim 12, wherein the disintegrant is a mixture of crospovidone having an average particle size within the range of 10 $\mu$m to 60 $\mu$m and crospovidone having an average particle size within the range of 80 $\mu$m to 200 $\mu$m.

14. The composition of claim 13, wherein the disintegrant is a mixture of 1 part by weight of crospovidone having an average particle size of 10 to 60 $\mu$m and 1 to 5 parts by weight of crospovidone having an average particle size of 80 to 200 $\mu$m.

15. The composition of any one of the preceding claims, wherein the core additionally comprises a lubricant selected from stearic acid, a hydrogenated vegetable oil, paraffin or an alkali or alkaline earth stearate, aluminum stearate, sodium stearyl fumarate or a mixture of two or more of these compounds, preferably magnesium or calcium stearate.

16. The composition of any one of the preceding claims, wherein the core additionally comprises a binder selected from microcrystalline cellulose, hydroxypropyl methyl cellulose, polyvinyl pyrrolidone or a mixtures of two or more of these compounds.

17. The composition of any one of the preceding claims, wherein the core comprises

15 to 50 % by weight of the active ingredient,
2 to 25 % by weight of the alkalizing substance,
10 to 50 % by weight of the filler, and
10 to 50 % by weight of the disintegrant.

18. The composition of any one of the preceding claims, wherein the optional separating layer comprises a water-soluble film- forming substance, preferably hydroxypropyl methyl cellulose.

19. The composition of any one of the preceding claims, wherein the optional separating layer has a thickness of 20 $\mu$m to 120 $\mu$m.

20. The composition of any one of the preceding claims, wherein the enteric coating comprises film-forming substances, preferably cellulose acetate phthalate, polyvinyl- acetate phthalate, hydroxypropyl-methyl cellulose phthalate, hydroxypropyl-methyl cellulose acetate succinate, ethylacrylate methacrylic acid copolymer, methylmethacrylate methacrylic acid copolymer, shellac, preferably copolymers of methacrylic acid and methacrylic acid esters such as anionic polymer of methacrylic acid and methacrylates with -COOH groups.

21. The composition of claim 1 wherein the tablet comprises 20 to 40 mg of pantoprazole in the form of sodium salt hydrate or sodium salt hydrate sesquihydrate.

**Patentansprüche**

1. Pharmazeutische Zusammensetzung, die Pantoprazol als Wirkstoff enthält und die die Form einer Tablette aufweist, die (a) einen Kern, der den Wirkstoff, Alkalisierungssubstanz Füllstoff und Sprengmittel enthält,

(b) eine optionale wasserlösliche Trennschicht und
(c) einen enterischen Überzug umfasst,

**dadurch gekennzeichnet, dass** die enterische Überzugsschicht eine Dicke von 85 $\mu$m bis 150 $\mu$m aufweist und

das Pantoprazol in der Form von Teilchen vorliegt, die eine durchschnittliche Teilchengröße im Bereich von 60 $\mu$m bis 250 $\mu$m aufweisen, wobei die Teilchen aus Pantoprazol bestehen und mittels eines Verfahrens erhalten werden können, bei dem man schrittweise Pantoprazol mit Wasser in einem Mischer mit hoher Scherkraft befeuchtet, man das feuchte Pantoprazol durch ein Sieb passiert, das eine Maschengröße im Bereich von 0,8 bis 2 mm aufweist, und man die Masse in einem Wirbelschichttrockner trocknet, wobei die Reihenfolge des Sieb- und des Trockenschrittes beliebig ist.

2. Pharmazeutische Zusammensetzung nach Anspruch 1, bei der die Pantoprazolteilchen eine durchschnittliche Teilchengröße im Bereich von 100 $\mu$m bis 250 $\mu$m aufweisen.

3. Zusammensetzung nach Anspruch 1 oder 2, bei der die Pantoprazolteilchen eine durchschnittliche Teilchengröße im Bereich von 125 $\mu$m bis 230 $\mu$m aufweisen.

4. Zusammensetzung nach einem der vorhergehenden Ansprüche, bei der die Pantoprazolteilchen eine durchschnittliche Teilchengröße im Bereich von 140 $\mu$m bis 200 $\mu$m aufweisen.

5. Zusammensetzung nach einem der vorhergehenden Ansprüche, bei der das Pantoprazol in Form des Natriumsalzes vorliegt.

6. Zusammensetzung nach Anspruch 5, bei der das Pantoprazol in der Form von Pantoprazol-Natrium-Sesquihydrat vorliegt.

7. Zusammensetzung nach einem der vorhergehenden Ansprüche, bei der das Alkalisierungsmittel ein Alkali- oder Erdalkalihydroxid, ein Alkali- oder Erdalkalicarbonat, ein Alkali- oder Erdalkalihydrogencarbonat, ein Phosphat, ein Glucosamin oder eine Mischung von zwei oder mehreren dieser Verbindungen ist, bevorzugt Natriumhydroxid, Natriumcarbonat, Natriumhydrogencarbonat, Natriumphosphat oder eine Mischung von zwei oder mehreren dieser Verbindungen, besonders bevorzugt Natriumcarbonat.

8. Zusammensetzung nach einem der vorhergehenden Ansprüche, bei der der Füllstoff Laktose oder ein Zuckeralkohol ist, wie Mannitol, Sorbitol, Maltitol, Erythrit oder eine Mischung von zwei oder mehreren dieser Verbindungen.

9. Zusammensetzung nach Anspruch 8, bei der der Füllstoff eine Mischung von Mannitol und Sorbitol ist.

10. Zusammensetzung nach Anspruch 9, bei der die Zusammensetzung 1 Gewichtsanteil Mannitol und 1 bis 5 Gewichtsanteile Sorbitol enthält, bevorzugt 2 bis 4 Gewichtsanteile Sorbitol, besonders bevorzugt 2.8 bis 3.8 Gewichtsanteile Sorbitol.

11. Zusammensetzung nach einem der Ansprüche 8 bis 10, bei der der Zuckeralkohol eine durchschnittliche Teilchengröße im Bereich von 30 $\mu$m bis 500 $\mu$m aufweist.

12. Zusammensetzung nach einem der vorhergehenden Ansprüche, bei der das Sprengmittel Crospovidon, Natriumstärkeglycolat, Croscarmellose-Natrium, Natriumcarboxymethylstärke, Natriumcarboxymethylcellulose, Kaliumcarboxymethylcellulose oder eine Mischung von zwei oder mehreren dieser Verbindungen ist.

13. Zusammensetzung nach Anspruch 12, bei der das Sprengmittel eine Mischung von Crospovidon, das eine durchschnittliche Teilchengröße im Bereich von 10 $\mu$m bis 60 $\mu$m aufweist, und Crospovidon ist, das eine durchschnittliche Teilchengröße im Bereich von 80 $\mu$m bis 200 $\mu$m aufweist.

14. Zusammensetzung nach Anspruch 13, bei der das Sprengmittel eine Mischung von 1 Gewichtsanteil Crospovidon, das eine durchschnittliche Teilchengröße im Bereich von 10 $\mu$m bis 60 $\mu$m aufweist, und 1 bis 5 Gewichtsanteilen Crospovidon ist, das eine durchschnittliche Teilchengröße im Bereich von 80 $\mu$m bis 200 $\mu$m aufweist.

15. Zusammensetzung nach einem der vorhergehenden Ansprüche, bei der der Kern zusätzlich ein Schmiermittel enthält, das aus Stearinsäure, hydriertem Pflanzenöl, Paraffin oder einem Alkali- oder Erdalkalistearat, Aluminiumstearat, Natriumstearylfumarat oder einer Mischung von zwei oder mehreren dieser Verbindungen, bevorzugt aus Magnesium- oder Calciumstearat ausgewählt ist.

16. Zusammensetzung nach einem der vorhergehenden Ansprüche, bei der der Kern zusätzlich ein Bindemittel enthält, das aus mikrokristalliner Cellulose, Hydroxypropylmethylcellulose, Polyvinylpyrrolidon oder einer Mischung von zwei oder mehreren dieser Verbindungen ausgewählt ist.

17. Zusammensetzung nach einem der vorhergehenden Ansprüche, bei der der Kern

15 bis 50 Gew.-% des Wirkstoffes,
2 bis 25 Gew.-% der Alkalisierungssubstanz,
10 bis 50 Gew.-% des Füllstoffes und
10 bis 50 Gew.-% des Sprengmittels enthält.

18. Zusammensetzung nach einem der vorhergehenden Ansprüche, bei der die optionale Trennschicht eine wasserlösliche filmbildende Substanz enthält, bevorzugt Hydroxypropylmethylcellulose.

19. Zusammensetzung nach einem der vorhergehenden Ansprüche, bei der die optionale Trennschicht eine Dicke von 20 $\mu$m bis 120 $\mu$m aufweist.

20. Zusammensetzung nach einem der vorhergehenden Ansprüche, bei der der enterische Überzug filmbildende Substanzen enthält, bevorzugt Celluloseacetatphthalat, Polyvinylacetatphthalat, Hydroxypropylmethylcellulosephthalat, Hydroxypropylmethylcelluloseacetatsuccinat, Ethylacrylatmethacrylsäure-Copolymer, Methylmethacrylatmethacrylsäure-Copolymer, Schellack, bevorzugt Copolymere von Methacrylsäure und Methacrylsäureestern, wie anionisches Polymer von Methacrylsäure und Methacrylaten mit -COOH Gruppen.

21. Zusammensetzung nach Anspruch 1, bei der die Tablette 20 bis 40 mg Pantoprozol in der Form von Natriumsalz Hydrat oder Natriumsalz Hydrat Sesquihydrat enthält.

## Revendications

1. Composition pharmaceutique comprenant du pantoprazole en tant que le composant actif, ayant la forme d'un comprimé comprenant

(a) un noyau comprenant le composant actif, une substance d'alcalinisation, un excipient et un désintégrant ;
(b) un couche optionnelle de séparation hydrosoluble ; et
(c) un enrobage entérique,

**caractérisée en ce que** la couche d'enrobage entérique a une épaisseur de 85 $\mu$m à 150 $\mu$m et **en ce que** le pantoprazole est présent sous la forme de particules ayant une taille moyenne de particule dans la plage de 60 $\mu$m à 250 $\mu$m, lesquelles particules consistent en pantoprazole et peuvent être obtenues par un procédé comprenant les étapes consistant à mouiller le pantoprazole avec de l'eau dans un mélangeur à fort cisaillement, faire passer le pantoprazole humide à travers un tamis ayant une taille de maille dans la plage de 0,8 à 2 mm, et sécher la masse dans un sécheur à lit fluidisé, l'ordre de l'étape de tamisage et de l'étape de séchage étant optionnel.

2. Composition pharmaceutique selon la revendication 1, dans laquelle les particules de pantoprazole ont une taille moyenne de particule dans la plage de 100 $\mu$m à 250 $\mu$m.

3. Composition selon la revendication 1 ou 2, dans laquelle les particules de pantoprazole ont une taille moyenne de particule dans la plage de 125 $\mu$m à 230 $\mu$m.

4. Composition selon l'une quelconque des revendications précédentes, dans laquelle les particules de pantoprazole ont une taille moyenne de particule dans la plage de 140 $\mu$m à 200 $\mu$m.

5. Composition selon l'une quelconque des revendications précédentes, dans laquelle le pantoprazole est présent sous forme du sel de sodium.

6. Composition selon la revendication 5, dans laquelle le pantoprazole est présent sous la forme de pantoprazole sodique sesquihydrate.

7. Composition selon l'une quelconque des revendications précédentes, dans laquelle l'agent d'alcalinisation est un hydroxyde de métal alcalin ou de métal alcalino-terreux, un carbonate de métal alcalin ou de métal alcalino-terreux, un hydrogénocarbonate de métal alcalin ou de métal alcalino-terreux, un phosphate, une glucosamine ou un mélange de deux ou plus de deux de ces composés, de préférence l'hydroxyde de sodium, le carbonate de sodium, l'hydrogénocarbonate de sodium, le phosphate de sodium ou un mélange de deux ou plus de deux de ces composants, de façon plus particulièrement préférée le carbonate de sodium.

8.  Composition selon l'une quelconque des revendications précédentes, dans laquelle l'excipient est le lactose ou un alcool glucidique, tel que le mannitol, le sorbitol, le maltitol, l'érythrol ou un mélange de deux ou plus de deux de ces composants.

9.  Composition selon la revendication 8, dans laquelle l'excipient est un mélange de mannitol et de sorbitol.

10. Composition selon la revendication 9, dans laquelle le mélange comprend 1 partie en poids de mannitol et 1 à 5 parties en poids de sorbitol, de préférence 2 à 4 parties en poids de sorbitol et de façon plus particulièrement préférée 2,8 à 3,8 parties en poids de sorbitol.

11. Composition selon l'une quelconque des revendications 8 à 10, dans laquelle l'alcool glucidique a une taille moyenne de particule dans la plage de 30 $\mu$m à 500 $\mu$m.

12. Composition selon l'une quelconque des revendications précédentes, dans laquelle le désintégrant est la crospovidone, le glycolate d'amidon sodique, la croscarmellose sodique, la carboxyméthylamidon sodique, la carboxyméthylcellulose sodique, la carboxyméthylcellulose potassique ou un mélange de deux ou plus de deux de ces composants.

13. Composition selon la revendication 12, dans laquelle le désintégrant est un mélange de crospovidone ayant une taille moyenne de particule dans la plage de 10 $\mu$m à 60 $\mu$m et de crospovidone ayant une taille moyenne de particule dans la plage de 80 $\mu$m à 200 $\mu$m.

14. Composition selon la revendication 13, dans laquelle le désintégrant est un mélange de 1 partie en poids de crospovidone ayant une taille moyenne de particule dans la plage de 10 $\mu$m à 60 $\mu$m et 1 à 5 parties en poids de crospovidone ayant une taille moyenne de particule dans la plage de 80 à 200 $\mu$m.

15. Composition selon l'une quelconque des revendications précédentes, dans laquelle le noyau comprend en plus un lubrifiant choisi parmi l'acide stéarique, une huile végétale hydrogénée, la paraffine ou un stéarate de métal alcalin ou de métal alcalino-terreux, le stéarate d'aluminium, le stéarylfumarate de sodium ou un mélange de deux ou plus de deux de ces composés, de préférence le stéarate de magnésium ou de calcium.

16. Composition selon l'une quelconque des revendications précédentes, dans laquelle le noyau comprend en plus un liant choisi parmi la cellulose microcristalline, l'hydroxypropylméthylcellulose, la polyvinylpyrrolidone ou un mélange de deux ou plus de deux de ces composés.

17. Composition selon l'une quelconque des revendications précédentes, dans laquelle le noyau comprend

15 à 50 % en poids du composant actif,
2 à 25 % en poids de la substance alcaline,
10 à 50 % en poids de l'excipient et
10 à 50 % en poids du désintégrant.

18. Composition selon l'une quelconque des revendications précédentes, dans laquelle la couche optionnelle de séparation comprend une substance filmogène hydrosoluble, de préférence l'hydroxypropylméthylcellulose.

19. Composition selon l'une quelconque des revendications précédentes, dans laquelle la couche optionnelle de séparation a une épaisseur de 20 $\mu$m à 120 $\mu$m.

20. Composition selon l'une quelconque des revendications précédentes, dans laquelle l'enrobage entérique comprend des substances filmogènes, de préférence de l'acétate-phtalate de cellulose, du poly(acétate-phtalate de vinyle), du phtalate d'hydroxypropylméthylcellulose, de l'acétate-succinate d'hydroxypropylméthyl-cellulose, du copolymère acrylate d'éthyle/acide méthacrylique, du copolymère méthacrylate de méthyle/acide méthacrylique, de la gomme-laque ; de préférence des copolymères d'acide méthacrylique et d'esters d'acide méthacrylique, tels qu'un polymère anionique d'acide méthacrylique et de méthacrylates comportant des groupes -COOH.

21. Composition selon la revendication 1, dans laquelle le comprimé comprend 20 à 40 mg de pantoprazole sous la forme de sel sodique hydrate ou de sel sodique hydrate sesquihydrate.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0166287 A
- EP 0244380 A
- WO 9222284 A
- WO 2005051348 A

**Non-patent literature cited in the description**

- **KOHL et al.** *The Journal of Medicinal Chemistry,* 1992, vol. 35 (6), 1049-1057